Europäisches Patentamt

**European Patent Office**

**Office européen des brevets**

(11) Publication number: **0 117 657**
**A1**

(12) **EUROPEAN PATENT APPLICATION**

(21) Application number: **84300627.1**

(22) Date of filing: **01.02.84**

(51) Int. Cl.³: **C 07 C 103/52, C 12 N 15/00, C 12 P 21/00, C 12 N 7/00, A 61 K 39/205**

(30) Priority: **01.02.83 US 462747**

(43) Date of publication of application: **05.09.84**
**Bulletin 84/36**

(84) Designated Contracting States: **AT BE CH DE FR GB IT LI LU NL SE**

(71) Applicant: **GENENTECH, INC., 460 Point San Bruno Boulevard, South San Francisco California 94080 (US)**

(72) Inventor: **Goeddel, David Vannorman, 2115 Forestview, Hillsborough California 94010 (US)**
Inventor: **Yelverton, Elizabeth McLeod, 1624 Danromas Way, San Jose California 95129 (US)**

(74) Representative: **Armitage, Ian Michael et al, MEWBURN ELLIS & CO. 2/3 Cursitor Street, London EC4A 1BQ (GB)**

(54) **Rabies immunogenic polypeptides and vaccine using them, DNA sequences, expression vectors, recombinant cells, and cultures therefor, and processes for their production.**

(57) Polypeptides which correspond to or are analogs of the glycoprotein associated with rabies virus, are synthesized by recombinant DNA technology, thereby poroviding noninfectious agents for use as rabies vaccine.

RABIES IMMUNOGENIC POLYPEPTIDES AND
VACCINE USING THEM, DNA SEQUENCES,
EXPRESSION VECTORS, RECOMBINANT CELLS,
AND CULTURES THEREFOR, AND PROCESSES
FOR THEIR PRODUCTION

Field of the Invention

This invention relates to polypeptides which correspond to or are analogs of the glycoprotein associated with rabies virus, and to the synthesis of said polypeptides by recombinant DNA technology. It further relates to noninfectious agents for use as rabies vaccine.

Background of the Invention

A. Rabies Virus Glycoprotein

The disease rabies is caused by a promiscuous neutrotropic virus whose hosts include all warm-blooded animals including, of course, humans. Virus particles, normally transmitted to the host by the bite of an infected animal, invade the nerve endings and travel via the spinal cord to the brain where they multiply and infect efferent neurons, including those supplying the salivary glands. The disease has been greatly feared since antiquity because it nearly always proves fatal to humans once symptoms develop. Dread of the disease is heightened because of the bizarre behavior induced in its victims which includes convulsions, raging, drooling and hydrophobia.

In 1885, Louis Pasteur developed the first rabies vaccine by emulsifying dessicated spinal cords of rabies-infected rabbits.

0258L

Treatment with the vaccine required that the victim/patient endure a series of painful abdominal injections of material extracted from cords which had been air-dried for periods of 15 days decreasing to five days.

The treatment today is less fearful to contemplate largely due to the efforts of workers at the Wistar Institute in Philadelphia, Pa. who developed a process whereby rabies virus can be cultured in vitro, concentrated, and inactivated to produce a more potent and more easily tolerated vaccine. The empirically -determined treatment regimen for exposed humans now includes injection with vaccine and anti-rabies immunoglobulin sometimes supplemented with interferon.

Despite these advances, the incidence of rabies worldwide is still high in hard-to-reach animal reservoirs, and a safe and effective synthetic vaccine has long been needed. In North America, mandatory vaccination programs have nearly eradicated canine rabies, but the disease is still widespread among the sylvatic population, particularly in skunks. In South America, the death of cattle infected by the bites of rabid vampire bats results in an estimated yearly loss of more $29,000,000. Since the 1940's. the incidence of fox rabies has been increasing in Europe, where a test program to vaccinate foxes orally by means of chicken heads containing attenuated rabies virus has been instituted.

Most present-day rabies vaccines are still made from inactivated or attenuated virus prepared from nerve tissue or avian embryos. Because of the possibility that an attenuated virus may revert to virulence, an oral virus-free vaccine would clearly be preferable.

The rabies virus itself is an RNA virus of the Lyssavirus genus in the Rhabdoviridae family. Some rabies virus strains, such as CVS, HEP, ERA, and PM, have been developed by cultivation in the laboratory. Since conventionally prepared, polyspecific antisera do not differentiate these strains, rabies virus has been considered to consist of a single antigenic species. Correspondingly, all commercially available rabies vaccines now are produced from single

0258L

strains. However, the higher resolution afforded by analysis with monoclonal antibodies has resulted in some variations among the strains being discerned. Also variations in the electrophoretic mobilities of some of the gene products have been observed. Five rabies viral proteins (polypeptides) have been identified from rabies virus-infected cells and from purified rabies virions: L (large, the viral polymerase), N (nucleocapsid protein), G (glycoprotein) and $M_1$ and $M_2$ (matrix proteins). Of these, the glycoprotein (or G protein) of about 60,000 daltons forms the external surface of the virus and is responsible for its immunogenicity; that is, antibodies raised against purified rabies virus glycoprotein can nuetralize infectious rabies virus. In fact, it has been shown by Dietzschold et al, Dev. Biol. Stand., 40, 45 (1978), that 9 ng of purified glycoprotein was equivalent to 1630 ng of purified whole virus in a mouse protection test even though the glycoprotein constitutes only about 40 percent of the virion protein. The amino acid sequences of the glycoproteins of the CVS and ERA rabies virus strains have now been deduced from DNA copies of the respective genes. See Anilionis et al, Nature, 284, 275 (1981). The homology (about 90 percent at the amino acid level) is in conformity with the closely related antigenic characteristics of the two virus strains. We perceived, therefore, that a subunit vaccine consisting of a glycoprotein gene product (or analog thereof) from a single rabies virus strain should protect against all, or nearly all, strains of infectious rabies virus and that the application of recombinant DNA technology would be a most effective way of providing the requisite large quantities of high quality immunogenic polypeptide separate from the other constituents of the virus associated with its infectious forms.

B.  Recombinant DNA Technology

Recombinant DNA technology has reached the age of some sophistication. Molecular biologists are able to recombine various DNA sequences with some facility, creating new DNA entities capable of producing copious amounts of exogenous protein product in

0258L

transformed microbes and cell cultures. The general means and methods are in hand for the in vitro ligation of various blunt ended or "sticky" ended fragments of DNA, producing potent expression vectors useful in transforming particular organisms, thus directing their efficient synthesis of desired exogenous product. However, on an individual product basis, the pathway remains tortuous and the science has not advanced to a stage where regular predictions of success can be made. Indeed, those who portend successful results without the underlying experimental basis, do so at considerable risk of inoperability.

DNA recombination of the essential elements, i.e., an origin of replication, one or more phenotypic selection characteristics, an expression promoter, heterologous gene insert and remainder vector, generally is performed outside the host cell. The resulting recombinant replicable expression vector, or plasmid, is introduced into cells by transformation and large quantities of the recombinant vehicle obtained by growing the transformant. Where the gene is properly inserted with reference to portions which govern the transcription and translation of the encoded DNA message, the resulting expression vector is useful to produce the polypeptide sequence for which the inserted gene codes, a process referred to as "expression." The resulting product may be obtained by lysis, if necessary, of the host cell and recovery of the product by appropriate purifications from other proteins.

In practice, the use of recombinant DNA technology can express entirely heterologous polypeptides--so-called direct expression--or alternatively may express a heterologous polypeptide fused to a portion of the amino acid sequence of a homologous polypeptide. In the latter cases, the intended bioactive product is sometimes rendered bioinactive within the fused, homologous/ heterologous polypeptide until it is cleaved in an extracellular environment.

Similarly, the art of cell or tissue cultures for studying genetics and cell physiology is well established. Means and methods are in hand for maintaining permanent cell lines, prepared by successive serial transfers from isolated normal cells. For use in research, such cell lines are maintained on a solid support in liquid medium, or by growth in suspension containing support nutriments. Scale-up for large preparations seems to pose only mechanical problems.

Likewise, protein biochemistry is a useful, indeed necessary, adjunct in biotechnology. Cells producing the desired protein also produce hundreds of other proteins, endogenous products of the cell's metabolism. These contaminating proteins, as well as other compounds, if not removed from the desired protein, could prove toxic if administered to an animal or human in the course of therapeutic treatment with desired protein. Hence, the techniques of protein biochemistry come to bear, allowing the design of separation procedures suitable for the particular system under consideration and providing a homogeneous product safe for intended use. Protein biochemistry also proves the identity of the desired product, characterizing it and ensuring that the cells have produced it faithfully with no alterations or mutations. This branch of science is also involved in the design of bioassays, stability studies and other procedures necessary to apply before successful clinical studies and marketing can take place.

Summary of the Invention

The present invention is based upon our discovery that recombinant DNA technology can be used to produce a polypeptide comprising the amino acid sequence, or a portion thereof, of the non-infectious glycoprotein associated with rabies virus which polypeptide demonstrates at least a substantial portion of the immunogenic activity of the glycoprotein obtained from the virus itself. The polypeptide can be produced directly or as a fusion protein in large amounts easily sufficient for use as an anti-rabies vaccine and at a much lower cost than would be required for isolation from native viruses. Being produced as a heterologous protein, the polypeptide is essentially free of other proteins of viral origin, and is free of the infectious agents associated with

0258L

the virus itself and, therefore, can be used as a vaccine without fear that the disease itself may be transmitted as can occur with the attenuated viruses used to make vaccines according to presently used techniques. Furthermore, these polypeptides are free of other protein material of mammalian origin.

The present invention, therefore, comprises an immunogenic polypeptide having an amino acid sequence which corresponds to at least a portion of the sequence of the glycoprotein associated with rabies virus, which polypeptide displays a degree of immunogenicity sufficient to stimulate production of anti-rabies antibodies which react with the glycoprotein when administered to a host from a species susceptible to rabies infection. All such species are mammalian.

The invention is further directed to replicable DNA expression vectors which contain gene sequences which encode for the polypeptide in expressible form. The invention is also directed to recombinant host cells such as microorganism strains or cell lines transformed with the expression vectors described above, and to the cultures thereof. Still further, the invention is directed to compositions comprising the polypeptide and particularly compositions useful as a rabies vaccine, and to the use of such vaccines to immunize mammals against rabies.

Accordingly, an object of the present invention is preparation of a polypeptide useful as a vaccine for rabies.

Another object is the preparation of a polypeptide for use as a vaccine for rabies which preparation is inexpensive relative to currently available techniques.

Yet another object is preparation of a rabies vaccine which does not contain any actual or potentially rabies infectious agents.

Brief Description of the Drawings

Figure 1 shows the deduced amino acid sequence of a rabies glycoprotein associated with the control virus standard (CVS) strain.

0258L

Figure 2 shows a portion of the steps in the construction of plasmids bearing gene sequences which permit expression of polypeptides-having-amino-acid-sequences corresponding to sequences of the protein shown in Figure 1.

Figure 3 shows the polyacrylamide slab gel analysis of cellular proteins from E. coli bearing plasmids which express polypeptides having amino acid sequences corresponding to sequences of the protein shown in Figure 1.

Detailed Description

A. Host Cell Cultures and Vectors

As used in the present application, the term "expression vector" includes vectors which are capable of expressing DNA sequences contained therein, where such sequences are operably linked to other sequences capable of effecting their expression. It is implied, although not always explicitly stated, that these expression vectors must be replicable in the host organisms either as episomes or as an integral part of the chromosomal DNA. Clearly a lack of replicability would render them effectively inoperable. In sum, "expression vector: is given a functional definition, and any DNA sequence which is capable of effecting expression of a specified DNA code disposed therein is included in this term as it is applied to the specified sequence. In general, expression vectors of utility in recombinant DNA techniques are often in the form of "plasmids" which refer to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

Further as used herein, the term "recombinant host cells" refers to cells which have been transformed with vectors constructed using recombinant DNA techniques.

0258L

The vectors and methods disclosed herein are suitable for use in host cells over a wide range of prokaryotic and eukaryotic organisms.

In general, of course, prokaryotes are preferred for cloning of DNA sequences in constructing the vectors useful in the invention. For example, E. coli K12 strain 294 (ATCC No. 31446) is particularly useful. Other microbial strains which may be used include E. coli strains such as E. coli B, and E. coli X1776 (ATTC No. 31537). These examples are, of course, intended to be illustrative rather than limiting.

Prokaryotes may also be used for expression. The aforementioned strains, as well as E. coli W3110 (F⁻, λ⁻, prototrophic, ATTC No. 27325), bacilli such as Bacillus subtilus, and other enterobacteriaceae such as Salmonella typhimurium or Serratia marcesans, and various pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR 322, a plasmid derived from an E. coli species (Bolivar, et al., Gene 2: 95 (1977)). pBR322 contains genes for ampicillin and tetracycline resistance and thus provides easy means for identifying transformed cells. The pBR322 plasmid, or other microbial plasmid must also contain, or be modified to contain, promoters which can be used by the microbial organism for expression of its own proteins. Those promoters most commonly used in recombinant DNA construction include the β-lactamase (penicillinase) and lactose promoter systems (Chang et al, Nature, 275: 615 (1978); Itakura, et al, Science, 198: 1056 (1977); (Goeddel, et al Nature 281: 544 (1979)) and a tryptophan (trp) promoter system (Goeddel, et al, Nucleic Acids Res., 8: 4057 (1980); EPO Appl Publ No. 0036776). While these are the most commonly used, other microbial promoters have been discovered and utilized, and details concerning their nucleotide sequences have

0258L

been published, enabling a skilled worker to ligate them functionally with plasmid vectors (Siebenlist, et al, _Cell_ 20: 269 (1980)).

In addition to prokaryates, eukaryotic microbes, such as yeast cultures may also be used. _Saccharomyces_ _cerevisiae_, or common baker's yeast is the most commonly used among eukaryotic microorganisms, although a number of other strains are commonly available. For expression in _Saccharomyces_, the plasmid YRp7, for example, (Stinchcomb, et al, _Nature_, 282: 39 (1979); Kingsman et al, _Gene_, 7: 141 (1979); Tschemper, et al, _Gene_, 10: 157 (1980)) is commonly used. This plasmid already contains the _trp1_ gene which provides a selection marker for a mutant strain of yeast lacking the ability to grow in tryptophan, for example ATCC No. 44076 or PEP4-1 (Jones, _Genetics_, 85: 12 (1977)). The presence of the _trp1_ lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman, et al., _J. Biol._ _Chem._, 255: 2073 (1980)) or other glycolytic enzymes (Hess, et al, _J. Adv. Enzyme Reg._, 7: 149 (1968); Holland, et al, _Biochemistry_, 17: 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and

0258L

galactose utilization (Holland, ibid.). Any plasmid vector containing yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However interest has been greatest in vertebrate cells, and propogation of vertebrate cells in culture (tissue culture) has become a routine procedure in recent years [Tissue Culture, Academic Press, Kruse and Patterson, editors (1973)]. Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promoter located in front of the gene to be expressed, along with any necessary ribosome binding sites, RNA splice sites, polyadenylation site, and transcriptional terminator sequences. It will be understood that this invention, although described herein in terms of a preferred embodiment, should not be construed as limited to those sequences exemplified.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Fiers, et al, Nature, 273: 113 (1978) incorporated herein by reference. Smaller or larger SV40 fragments may also be used, provided there is included the approximately 250 bp sequence extending from the Hind III site toward the Bgl I site located in the viral origin of replication. Further, it is also possible, and often desirable, to utilize promoter or control sequences normally associated with the desired gene sequence, provide such control sequences are compatible with the host cell systems.

0258L

An origin of replication may be provided either by construction of the vector to include an exogenous origin, such as may be derived from SV40 or other viral (e.g. Polyoma, Adeno, VSV, BPV, etc.) source, or may be provided by the host cell chromosomal replication mechanism. If the vector is integrated into the host cell chromosome, the latter is often sufficient.

B. Methods Employed

If cells without formidable cell wall barriers are used as host cells, transfection is carried out by the calcium phosphate precipitation method as described by Graham and Van der Eb, Virology, 52: 546 (1978). However, other methods for introducing DNA into cells such as by nuclear injection or by protoplast fusion may also be used.

If prokaryotic cells or cells which contain substantial cell wall constructions are used, the preferred method of transfection is calcium treatment using calcium chloride as described by Cohen, F.N. et al Proc. Natl. Acad. Sci. (USA), 69: 2110 (1972).

Construction of suitable vectors containing the desired coding and control sequences employ standard ligation techniques. Isolated plasmids or DNA fragments are cleaved, tailored, and religated in the form desired to form the plasmids required.

Cleavage is performed by treating with restriction enzyme (or enyzmes) in suitable buffer. In general, about 1 µg plasmid or DNA fragments is used with about 1 unit of enzyme in about 20 µl of buffer solution. (Appropriate buffers and substrate amounts for particular restriction enzymes are specified by the manufacturer.) Incubation times of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 15 minutes at 15° with 10 units of E. coli DNA Polymerase I (Klenow), phenol-chloroform extracted, and ethanol precipitated.

0258L

-12-

Size separation of the cleaved fragments is performed using 6 percent polyacrylamide gel described by Goeddel, D., et al, Nucleic Acids Res., 8: 4057 (1980) incorporated herein by reference.

For ligation approximately equimolar amounts of the desired components, suitably end tailored to provide correct matching are treated with about 10 units T4 DNA ligase per 0.5 μg DNA. (When cleaved vectors are used as components, it may be useful to prevent religation of the cleaved vector by pretreatment with bacterial alkaline phosphatase.)

The ligation mixture was used to transform E. coli K12 strain 294 (ATLC 31446), and successful transformants were selected by ampicillin resistance. Plasmids from the transformants were prepared, analyzed by restriction and/or sequenced by the method of Messing, et al, Nucleic Acids Res., 9:309 (1981) or by the method of Maxam, et al, Methods in Enzymology, 65:499 (1980).


C.  Preferred Embodiments

The following description of preferred embodiments involving polypeptide expression in E. coli is intended to illustrate but not to limit the invention.


C.1.  Determination of Structural Features of Rabies Virus Glycoprotein

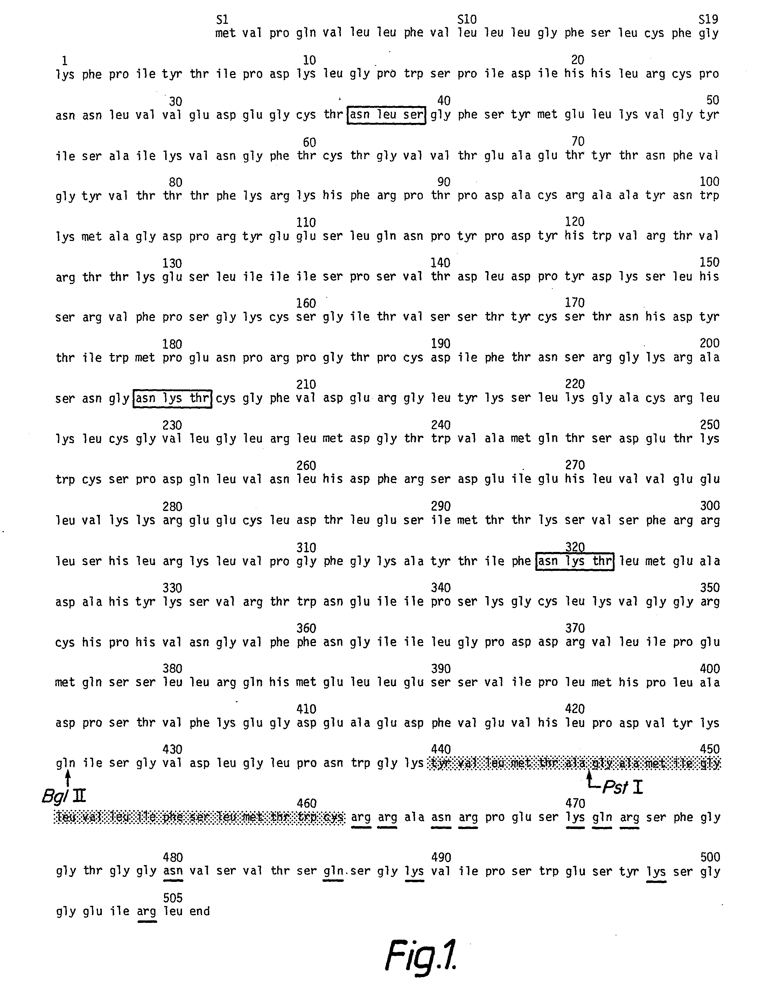

From polyadenylated mRNAs of rabies (CVS strain) virus-infected cells, we prepared double stranded complementary DNA (cDNA) species, introduced single stranded homopolymeric "tails," and annealed them via DNA base pairing into the bacterial cloning vehicle pBR322 described by F. Bolivar et al, Gene, 2, 95 (1977). The heterogeneous hybrid plasmids were then transformed into Escherichia coli to form a colony library. From the library we obtained a plasmid, designated by us pRab91, which selectively hybridized to rabies virus glycoprotein mRNA. Nucleotide sequencing of the 2100 basepair (bp) cloned cDNA showed that it encoded most of the hydrophobic signal peptide and all of the mature rabies virus glycoprotein, whose $NH_2$- and COOH- termini have been established by direct amino acid analysis. See Lai et al, Biochemical and Biophysical Research Communications, 103, 536 (1981). The amino acid sequence predicted from the cDNA is shown in Figure 1, where some features of the polypeptide are highlighted and more completely described in the Detailed Discussion of the Drawings hereinafter.

Although overall the rabies virus glycoprotein is quite hydrophobic, the signal peptide (denoted $S_1$-$S_{19}$) and the 22 amino acid (stippled) region between amino acid positions 439 and 462 are particularly hydrophobic. This latter domain has been postulated to constitute the portion of the protein which would span the viral lipid membrane. In that regard, it was satisfying to note that nine of these predicted 22 amino acid positions differ between the glycoproteins of the CVS and ERA rabies strains, but none of the changes alter the hydrophobicity. Hypothetically, the native immunogenic domains of the glycoprotein would occur $NH_2$-terminal to this transmembrane segment; the C-terminal portion would be the site of interaction with matrix protein. In Figure 1, the many charged residues which follow the hydrophobic segment are indicated by underlining. Three sites of possible sugar chain attachment, asn-x-thr or asn-x-ser which might occur on the virus exterior, are also shown; these are consistent in number with the most highly glycosylated form of the protein.

C.2. Preparation of Expression Vectors and Polypeptide Synthesis

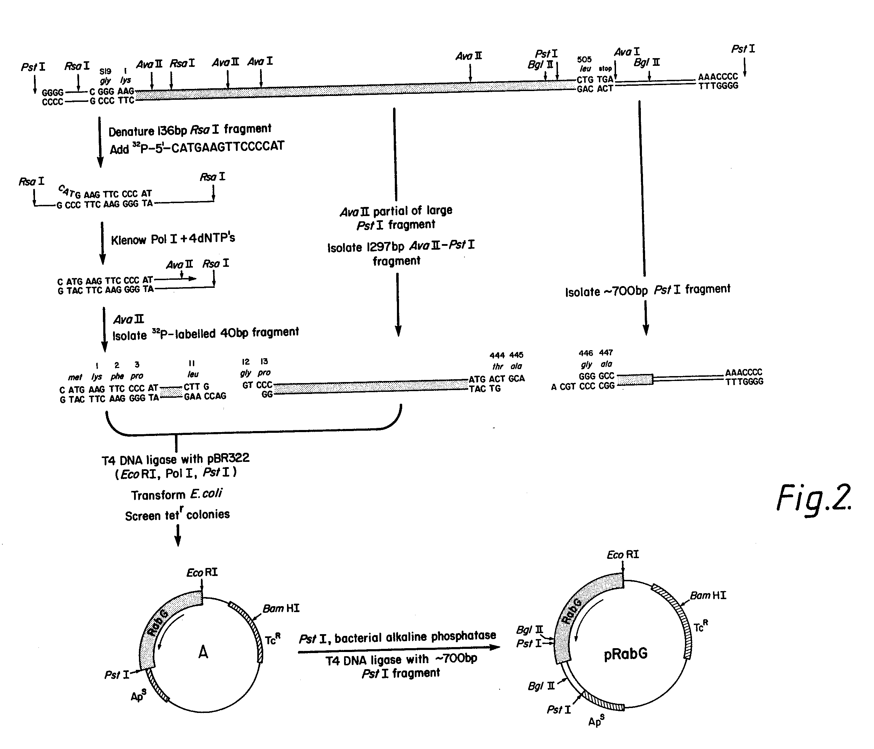

In order for us to obtain the expression of mature peptide in E. coli having an amino acid sequence corresponding to a sequence of rabies glycoprotein, it was determined to: 1) add a translation initiation signal before the codon for the $NH_2$-terminal amino acid, 2) place the gene downstream from an E. coli promoter, or site of initiation of transcription by RNA polymerase, and 3) make adjustments which would enable the E. coli host to

0258L

perform translation efficiently. The method for assembling the gene which could be inserted into appropriate expression vectors to accomplish these objectives is described below with reference to Figure 2. Additional details are provided in the Detailed Description of the Drawings.

### C.2.A. Construction of pRabG trp 1

The > 2000 bp DNA PstI fragment from the plasmid designated pRab91 was treated to obtain a 136 bp RsaI restriction fragment which contains the coding region for the signal peptide cleavage site. This fragment was denatured in the presence of a synthetic $^{32}$P labeled deoxyoligonucleotide primer 5'-dCATGAAGTTCCCCAT. This primer was selected to incorporate an ATG translation initiation codon before the codon "AAG" for lysine, the first amino acid of the mature rabies glycoprotein gene. The primer mismatches with the negative template strand in only two positions and includes a deoxycytidine residue at the 5' position for subsequent assembly to an EcoRI recognition site.

E. Coli DNA polymerase I large fragment was used to synthesize the revised sense strand, while at the same time removing the 3' protruding end and repairing mismatches in the minus strand. A 40 bp fragment was obtained by means of a second restriction site within the repair template which generated a defined AvaII sticky end. This fragment was ligated with the 1297 bp AvaII-PstI fragment obtained from the > 2000 bp DNA PstI fragment from plasmid pRab91, a fragment containing most of the rabies glycoprotein coding region.

The resulting 1337 bp fragment was propagated in a modified pBR322 plasmid which was prepared by opening the pBR322 plasmid at a unique EcoRI site, treating with DNA polymerase I in the presence of deoxyribonucleoside triphosphates to fill in the single stranded ends, and restricting again with PstI. Tetracycline resistant E. coli transformants were prescreened to select correct transformation products by colony hybridization with

0258L

-15-

the same $^{32}$P-labeled DNA primer referred to above.

The correct plasmid A as shown in Fig. 2 was then opened at the unique PstI site, treated with bacterial alkaline phosphatase to preclude self-ligation, and ligated in a molar excess of the approximately 700 bp PstI fragment bearing the remaining coding sequence for the rabies glycoprotein and the non-translated 3' end. Asymmetric AvaI sites were used to determine the orientation of insertion of the 700 bp fragment and it was determined that approximately one-half of the fragment had been inserted in a proper orientation to permit expression of the entire gene. The resulting plasmid, designated pRabG by us, is shown in Fig. 2.

As shown in Fig. 2, a unique EcoRI recognition site directly precedes the structural gene for mature rabies glycoprotein in pRabG. Advantage was taken of this site to obtain the regulatory elements for rabies glycoprotein gene expression by addition of a 300 bp EcoRI-bounded promoter cassette isolated from the plasmid pLeIFA25 described by D. V. Goeddel et al, Nature, 287, 411 (1980) containing the congruent promoter and repressor binding site (operator), and also the ribosome binding site for the E. coli tryptophan (trp) operon leader peptide described by Yanofsky et al, Nucleic Acids Research, 9, 6647 (1981).

The plasmid pRabG was opened by restriction with EcoRI, treated with bacterial alkaline phosphatase, and ligated with the 300 bp promoter fragment. The resulting ligation mix was used to transform competent E. coli 294 to tetracycline resistance. An asymmetrically located XbaI site in the EcoRI fragment was used to screen for transformant plasmids which had the promoter fragment in the orientation for expression of the rabies glycoprotein. A properly assembled plasmid was identified and designated pRabGtrp1. After the trp promoter fragment was added, the nucleotide sequence around the EcoRI junction was determined and showed that incorporation of the new translation initiation codon had occurred as planned.

0258L

C.2.B   Construction of pRabGT1

In an alternate order of assembly, the EcoRI promoter fragment was inserted prior to the addition of the approximately 700 bp PstI fragment bearing the rabies glycoprotein COOH-terminal coding sequence. In the resulting plasmid, designated by us pRabGT1, the rabies glycoprotein text is brought into phase at amino acid position 446 with the portion of the β-lactamase gene which extends from the PstI site of pBR322, which results in the addition of 103 more codons before a translation stop signal is reached.

C.2.C   Expression of Rabies G Sequences in pRabGtrp1 and RabGT1

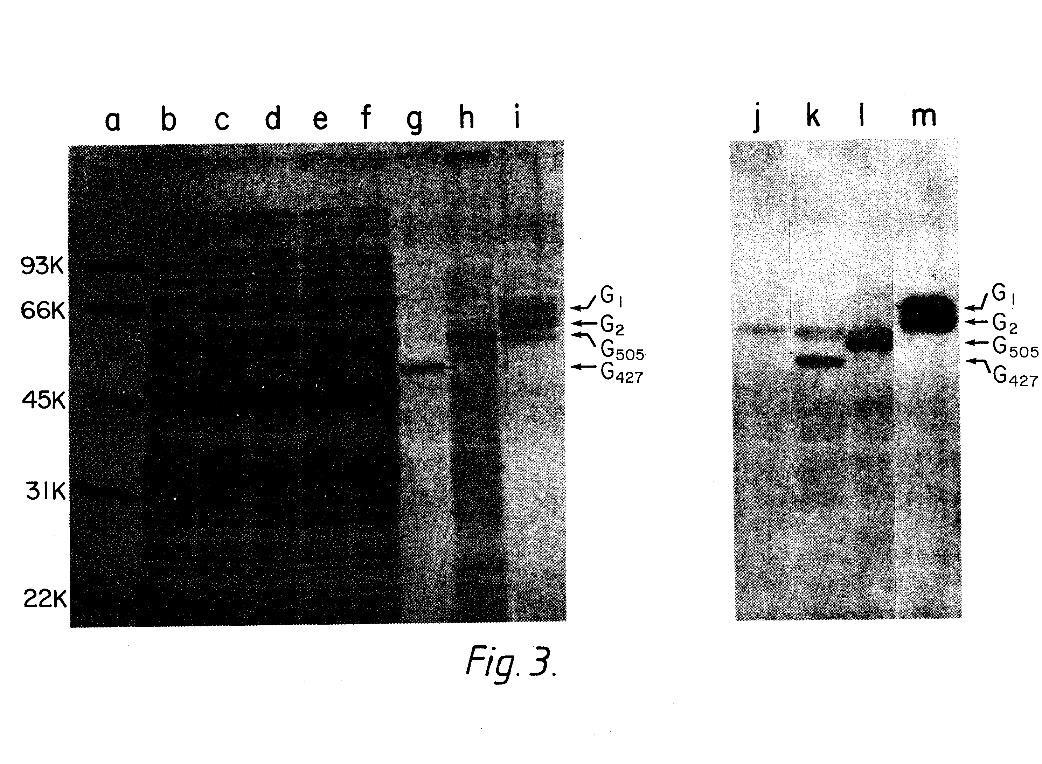

A preliminary test for expression of the rabies glycoprotein gene in E. coli was to examine by gel electrophoresis whole cell extracts of E. coli transformed by pRabGT1, pRabGtrp1, or a control plasmid pIFN-γtrp48. A major new protein band, with migration commensurate with a size of about 62,000 daltons, was readily visible by Coomassie-blue staining in the gel lane containing the extract of E. coli W3110 (F⁻,K-12 derivative described by B.J. Bachmann, Bac. Rev., 36, 525 (1972)) transformed with pRabGT1. This is shown in lane c of Figure 3, more details of which are contained in the Detailed Discussion of the Drawings hereinafter. The presence of the protein correlated with tryptophan depletion in the culture medium and it constituted about 3 percent of the total cell proteins. The protein expressed from pRabGtrp1 was not as easily detectable, however. Therefore, one further manipulation was made to the expression vector to increase production of rabies glycoprotein.

C.2.D   Construction of pRabdex2 and pRabdex31

The DNA fragment which was introduced to provide a promoter in pRabGtrp1 and pRabGT also supplies a recognitionsite for XbaI within the presumed ribosome binding site as follows:   (5') GGTATCTAGAATTCATG (the underlined nucleotides

0258L

constitute the XbaI site, and the barred nucleotides show the Shine-Dalgarno and translation initiation sequences).

The plasmids pRabGT1 and pRabGtrp1 were altered by restriction with XbaI, treatment with S1 nuclease, and religation with T4 DNA ligase, as described by H. M. Shepard et al, DNA, 1, 125 (1982). Plasmid DNA from transformants which arose on selective medium were screened for the loss of the XbaI recognition site. Plasmids which we designated pRabdex31 (derived from pRabGT1) and pRabdex2 (derived from pRabGtrp1) were isolated and determined to have lost this site by nucleotide sequencing which confirmed that the four nucleotides which form single stranded ends after XbaI scission had been removed to generate the new ribosome binding site GGTATAATTCATG.

C.2.E.   Expression of Rabies Sequences of pRabdex2 and pRabdex31

Cultures of E. coli W3110 transformed by pRabdex31 and E. coli W3110 transformed by pRabdex2 were grown in the absence of tryptophan. Overnight culture of either of the two strains in Luria broth plus 5 μg/ml tetracycline was used to innoculate M9 medium containing 0.2 percent glucose, 0.5 percent casamino acids and 5 μg/ml tetracycline at a 1:100 dilution. Indoleacrylic acid was added to 20 μg/ml when $A_{550}$ was between 0.1 and 0.2. The cells were harvested by centrifugation at $A_{550} = 1.0$ and extracted. In gel electrophoresis of these whole cell extracts (lanes d and f of Fig. 3), the full length bacterially expressed rabies glycoprotein, which we termed $G_{505}$, comigrated with a major class of E. coli proteins, but it can be observed as a widening of the stained protein band at about 57,000 daltons. Densitometry scanning of the electrophoretically separated E. coli W3110/pRabdex2 proteins, compared to control bacterial extract proteins, showed that $G_{505}$ constituted about 2 percent of the cellular proteins, while the amount of fusion protein expressed from pRabdex31 was slightly less than 5 percent.

0258L

C.2.F   Construction and Expression of pRabdexl

Figure 1 shows the location of a BglII restriction site which was used to design a truncated glycoprotein gene, which would encode a form of the glycoprotein which lacks the hydrophobic region designated by the shaded portion of Figure 1. Inspection of the sequence in the 3'-untranslated portion of the rabies glycoprotein cDNA (not shown) revealed that excision of an internal BglII fragment from the plasmid pRabdex2 would result in a TAG triplet immediately after the codon for amino acid 427 of the mature rabies glycoprotein. The positions of the two BglII sites are indicated in Figure 2.

The plasmid pRabdex2 was restricted with BglII and religated with $T_4$ DNA ligase. The ligation mix was used to transform E. coli 294 (D.V. Goeddel et al, Proc. Natl. Acad. Sci., USA, 76, 106 (1979) and Goeddel et al, Nature, 281, 544 (1979) to tetracycline resistance; plasmid DNA from several isolates was screened for the loss of the internal BglII fragment. The plasmid pRabdexl met this criterion and was selected for further characterization. Gel electrophoresis of a whole cell extract of E. coli W3110/pRabdexl is shown in lane e of Figure 3. The truncated glycoprotein, which we termed $G_{427}$, can be observed readily at a position corresponding to about 49,000 daltons; it constitutes about two to three percent of the cell proteins. In Table 1, the estimated synthetic levels of $G_{505}$ and $G_{427}$ are shown in relation to the number of protein molecules per bacterium. The per cell synthesis of the more hydrophilic polypeptide, $G_{427}$, is about 1.5 fold higher than that of $G_{505}$, or nearly equal to the level of synthesis of the rabies glycoprotein-β-lactamase fusion protein.

C.3   Characterization of Rabies Glycoprotein Analogs

To demonstrate the antigenic identity of the proteins produced by expression from the plasmids pRabdexl and pRabdex2, cell extracts were electrophoresed on polyacrylamide slab gels as described hereinafter in the Detailed Discussion of the Drawings relating to Fig. 3, tranferred to nitrocellulose, and reacted in

0258L

situ with rabbit serum raised against authentic rabies glycoprotein, also as described hereinafter. Lanes j-m of Fig. 3 show the results of this procedure. We found that the antiserum used reacts somewhat with a normal E. coli component, as can be seen in lane j in which a control E. coli extract has been electrophoresed. The more intensely labeled bands in lanes k and l correspond with the rabies virus specific bands easily visible in stained slab gels. The gel from which this transfer was made is not shown, but is typified by extracts shown in lanes b, e, and f. The rabies glycoprotein-β-lactamase fusion gave a similar reaction but which is not shown.

Polyacrylamide gel analysis of proteins from rabies virions of the CVS strain normally discriminates two electrophoretic glycoprotein species, $G_1$ and $G_2$, which differ in their degree of glycosylation. See Dietzschold et al, Virology, 98, 63 (1979). Lane m of Figure 3 shows the $^{125}$I-labelled bands corresponding to the more slowly migrating authentic glycoproteins, $G_1$ and $G_2$. This procedure showed that at least some of the antigenic determinants survived the denaturing and reducing agents present in the gel loading buffer. Furthermore, it showed that at least some of the antigenic determinants are independent of postranslational modifications such as carbohydrates side chain attachment which are made on the authentic glycoprotein in rabies infected cells, but which would not be made in Escherichia coli.

Lai et al, Biochemical and Biophysical Research Communications, 103, 536 (1981) reported that in order to dissolve rabies glycoprotein for amino acid sequence determination it was necessary to boil protein preparations in 1 percent SDS. The bacterially produced glycoprotein analogs seem to be insoluble under ordinary conditions as well. We obtained an approximately 10-fold enrichment of $G_{427}$ and $G_{505}$ from cell extracts of E. coli W3110/pRabdex1 and E. coli W3110/pRabdex2, respectively, by collecting the precipitate of a lysozyme-0.2 percent NP40-1.5M NaCl lysate, as described in the detailed description of Fig. 3 concerning lanes g and h. Enriched preparations were then used to

0258L

assess the antigenicity of the proteins by comparison with authentic glycoprotein in an enzyme linked immunosorbent assay (ELISA).

For the ELISA, dilutions of either purified rabies glycoprotein or the test bacterial samples were bound to the surface of a plastic microtiter tray, and were reacted with high titer rabbit antiserum directed against authentic rabies glycoprotein. Unbound antibody was washed away, and the samples were treated with goat anti-rabbit antibody coupled to the enzyme alkaline phosphatase. Cleavage of the alkaline phosphatase substrate resulted in a yellow color development that was monitored spectrophotometrically. The conditions were set so that the absorbance response was linear in the concentration range of 50-500 ng/ml of rabies glycoprotein. The details of this ELISA procedure are given hereinafter.

When the insoluble protein fractions from cultures of E. coli W3110/pRabdex1 and E. coli W3110/pRabdex2 were assayed, it was found that the proteins $G_{427}$ and $G_{505}$ were about 2-3 percent as effective per weight as purified authentic glycoprotein in binding the anti-glycoprotein antibodies. To recover more activity from the bacterially synthesized proteins, a rather vigorous solubilization procedure was employed. The enriched preparations were resuspended by vortexing in 7M guanidine-hydrochloride at room temperature followed by dialysis into 7M urea, 5mM Tris pH 8.0. Results are improved when the solubilzation is carried out concurrently with conversion of the polypeptide to the corresponding sulfonate and inclusion of a suitable reducing agent with the dialysis mixture, basically as described in US Appl. Serial No. 452,187 (European Patent Application 83307840.5). Samples were diluted up to 1:2000 in phosphate buffered saline just prior to assay. Under these conditions, the proteins dissolved and their apparent activity increased over 30-fold in the ELISA. Freshly solubilized preparation of $G_{505}$ reacted with the antibody an average of about 90 percent as effectively as authentic glycoprotein, while the activities of solubilized preparations of $G_{427}$ were approximately 70 percent those of authentic

0258L

glycoprotein. This reconstitution of activity by solubilization showed that the low antigenicity levels first observed were not inherent properties of the bacterially synthesized glycoprotein analogs, $G_{505}$ and $G_{427}$.

C.4 ELISA Assay Procedure for Rabies Antigenicity C.4.A Preparation of Rabbit Serum Against Rabies Glycoprotein

Anti-rabies glycoprotein preparation was raised against the glycoprotein-containing NP-40 supernatant fraction from purified rabies virions. Rabbits were injected once and boosted at three weeks; serum titers (all 1:30,000 3 weeks after boost) were determined by immunofluorescent neutralizing antibody assay. A single crude serum sample from a single rabbit (titer 1:34,800) was diluted 1:300-1:1000 for use in ELISA or for filter hybridization. In some experiments, antiserum was preadsorbed to control bacterial extracts to eliminate nonspecific reaction in the ELISA. All antibody binding steps and washes were done in 50 mM Tris pH 7.4, 0.15M NaCl, 5mM EDTA, 0.25 percent NP-40 at room temperature for one-two hours.

C.4.B Details of ELISA Procedure

Sample antigens to be assayed were diluted into phosphate buffered saline (PBS) and 100 µl pipetted onto the wells of a Falcon flexible assay plate. After 30 minutes incubation, unbound sample was aspirated away, and 100 µl PBS + 0.25 percent gelatin was applied. After 15 minutes the gelatin solution was removed and te wells were washed three times with PBS + .05 percent Tween. Rabbit anti-rabies glycoprotein serum (100 ul) diluted in PBS-Tween was added and incubated for 30 minutes. After washing three times in PBS-Tween, 100 µl of goat anti-rabbit IgG-alkaline phosphatase conjugate was added (Zymed Laboratories; diluted 1:1000 in PBS-Tween just prior to use). After 30-60 minutes incubation and washing, 100 µl of substrate (P-nitrophenylphosphate, reconstituted according to Zymed instructions) was added. $A_{405}$

0258L

was determined on a Dynatech ELISA reader. The standard plot for each experiment was authentic rabies glycoprotein diluted in duplicate from 5 to 50 ng per well.

D.   Detailed Discussion of the Drawings

Figure 1. The deduced amino acid sequence of a rabies virus glycoprotein from the control virus standard (CVS) strain is shown. The first amino acids of the mature protein are indicated as determined by $NH_2$-terminal analysis of the glycoprotein from another rabies virus strain described by Lai et al, Biochemical and Biophysical Research Communications, 103, 536 (1981). The stippled portion between amino acids 439 and 462 is a hydrophobic domain which may be the site of membrane attachment. Carboxy-terminal to this charged residues which may interact with capsid proteins are underlined. The amino acid sequence contains four potential glycosylation sites (asn-X-ser or asn-X-thr). Those three that would hypothetically occur on the external portion of the glycoprotein are indicated by boxing. Two restriction enzyme recognition sites, which occur in the cDNA and were used in the construction of truncated or fused rabies glycoprotein analogs, are shown.

Figure 2. Construction of a plasmid bearing the gene for mature rabies glycoprotein. The plasmid pRab91, a pBR322 derivative with rabies glycoprotein cDNA introduced by dG/dC pairing at the PstI site was the source of the template for a DNA polymerase I (Klenow fragment) catalyzed repair reaction. The synthetic deoxynucleotide dCATGAAGTTCCCCAT, synthesized by the modified phosphotriester method of Crea et al, Nucleic Acids Research, 10, 3605 (1980) was phosphorylated in a reaction containing 500 pmol primer, ~300 µCi $[\gamma^{32}p]$-ATP (2,500 Ci/mmol, New England Nuclear) and 2 units T4 polynucleotide kinase in 60mM Tris-HCl (pH 8) 10mM $MgCl_2$, 15mM β-mercaptoethanol. The reaction was allowed to proceed for 20 minutes at 37°C, then unlabelled ATP was added to a final concentration of 200 µm. After 30 minutes the reaction was

0258L

terminated by phenol/chloroform extraction, and ethanol precipitated with 3 µg of the 136 bp template RsaI fragment. The mixture was dissolved in 50 µl water, boiled five minutes, quenched in dry ice, and diluted at 0°C to 100 µl to contain 10mM Tris-HCl (pH 7.5), 7mM MgCl$_2$, 60mM NaCl and 0.5mM each deoxyribonucleotide triphosphate. Ten units of DNA polymerase I Klenow fragment (Boehringer-Mannheim) were added and the reaction was allowed to proceed at ambient temperature for four hours. After phenol extraction and ethanol precipitation, the mixture was treated with 15 µl AvaII (New England Biolabs) using manufacturer's recommended conditions. The reaction mixture was electrophoresed through a 10 percent polyacrylamide slab gel; the desired 40 bp fragment was located by autoradiography, excised and electroeluted. Next, plasmid pRab91 was partially digested with AvaII, then digested to completion with PstI. A 1297 bp fragment was recovered after gel electrophoresis of the restriction products. This fragment was used in a three-part ligation using T4 DNA ligase with the radiolabelled 40 bp repaired fragment and with a modified pBR322 vector. The ligation mix was transformed into E. coli 294 with selection on LB (Luria broth) plates containing 5 µg ml$^{-1}$ tetracycline. Plasmid DNA of the transformants was screened by in situ hybridization with the radiolabelled synthetic oligonucleotide and then by digestion with restriction enzymes. The intermediate plasmid was then cleaved with PstI, treated with bacterial alkaline phosphatase, and ligated with the ~700 bp PstI fragment containing the remaining cDNA.

Figure 3. Discontinuous, 10 percent polyacrylamide slab gel analysis of cellular proteins from E. coli bearing rabies virus glycoprotein expression plasmids. Lane a contained molecular weight standard proteins purchased from Bio-Rad. Lanes b-f are total cell extracts of E. coli W3110 transformed with (b) a control plasmid, pIFN-γtrp48 (B. J. Bachmann, Bac. Rev., 36, 525 (1972); (c) pRabGT1, (d) pRabdex 31, (e) pRabdex 1, and (f) pRabdex 2. Inocula were grown overnight in LB + 5 µg ml$^{-1}$ tetracycline, then diluted 1:100 in M9 medium containing 0.2 percent glucose, 0.5 percent casamino

0258L

acids, and tetracycline. Indole acrylic acid was added to a concentration of 25 µg ml$^{-1}$ when $A_{550}$ reached 0.1. Cells were collected by centrifugation when $A_{550}$ reached 1.0. Total cell extracts were prepared by boiling in 2 percent sodium dodecyl sulfate and 0.1M β-mercaptoethanol. Lanes g and h show enriched preparations of G427 and G505, respectively. Five hundred ml cultures of E. coli W3110/pRabdex 1 and E. coli W3110/pRabdex2 were grown in tryptophan-depleted medium as above, harvested by centrifugation at $A_{550}$ = 1.0, and treated with lysozyme, 0.2 percent NP-40, and 1.5M NaCl essentially as described by Kleid et al, Science, 214, 1125 (1981). The insoluble pellet was slurried into phosphate buffered saline. Samples to be analyzed by gel electrophoresis were dissolved by boiling in buffer containing 20 percent glycerol, 5 percent β-mercaptoethanol. 0.125M Tris-HCl (pH 6.8) and 4 percent sodium dodecyl sulfate. Lane i shows a partially purified rabies virus glycoprotein prepared by NP-40 solubilization of rabies virions. Some faster migrating nucleocapsid (N) protein is also present in this preparation. All proteins in lanes a-i are visualized by staining with Coomassie brilliant blue. Lanes j-m show an autoradiogram of a "Western blot" analysis of E. coli-derived or authentic rabies glycoprotein. Total cell extracts of E. coli W3110 containing plasmids (j) pIFN-trp48, (k) pRadbex1, or (1) pRabdex2 were electrophoresed aside (m) authentic glycoprotein and transferred electrophoretically to nitrocellulose filter paper. The filter paper was treated serially with rabbit anti-rabies G serum and with $^{125}$I-labelled protein A from Amersham. K represents 1,000 Mr.

### E.1    Pharmaceutical Compositions

The foregoing description demonstrates that polypeptides can be obtained by recombinant DNA technology having amino acid sequences which conform to sequences in rabies glycoprotein and that the polypeptides exhibit substantial rabies glycoprotein antigenicity. Such polypeptides are useful as rabies

0258L

subunit vaccines and can be obtained readily at low cost and free of infectious agents associated with rabies serums.

The compounds of the present invention can be formulated according to known methods to prepare pharmaceutically useful compositions, whereby the polypeptide hereof is combined in admixture with a pharmaceutically acceptable carrier vehicle. Suitable vehicles and their formulation are described in Remington's Pharmaceutical Science by E.W. Martin, which is hereby incorporated by reference. Such compositions will contain an effective amount of the polypeptide product hereof together with a suitable amount of carrier vehicle in order to prepare pharmaceutically acceptable compositions suitable for effective administration to the host. One preferred mode of administration is parenteral. Suitable animal health formulations are prepared as in the case of pharmaceutical compositions, mutatis mutandis.

### E.2    Vaccine Preparation

The vaccines of the present invention, incorporating a suitable polypeptide produced as herein described, can be prepared according to known methods, wherein said polypeptide is combined in admixture with a suitable vehicle. Suitable vehicles include, for example, saline solutions, various known adjuvants, or other additives recognized in the art for use in compositions applied to prevent viral infections. Such vaccines will contain an effective amount of the polypeptide hereof and a suitable amount of vehicle in order to prepare a vaccine useful for effective administration to the host. Attention is also directed to New Trends and Developments in Vaccines, Editors: A. Voller and H. Friedman, University Park Press, Baltimore, 1978, which is hereby incorporated by reference, for further background details on the preparation of vaccines.

Because of the unique methods by which the active component of these vaccines are produced, vaccines hereof are likely to be substantially free of extraneous protein(s) and of other viral and cellular components. Therefore, they are less likely to produce

0258L

the complications of whole killed or attenuated virus vaccine preparations.

It will be apparent to those skilled in the art in the light of the foregoing discussion that the invention, such as in the selection of intended polypeptide product, is not to be limited to the preferred embodiments hereof but rather only to the lawful scope of the appended claims.

CLAIMS

1.  A polypeptide comprising the amino acid sequence of at least a portion of the non-infectious glycoprotein associated with rabies virus, which polypeptide is:

a)  essentially free of other protein material of viral origin; and

b)  immunogenic when administered to a mammal so as to elicit formation of antibodies which recognize rabies associated glycoprotein.

2.  The polypeptide of claim 1 which is unaccompanied by glycosylation.

3.  The polypeptide of claims 1 or 2 which is essentially free of proteins of mammalian origin.

4.  The polypeptide of claims 1, 2 or 3 which comprises a sequence of the 427 amino acids which are numbered 1 to 427 in Figure 1.

5.  The polypeptide of claims 1, 2 or 3 which comprises the sequence of 505 amino acids which are numbered 1 to 505 in Figure 1.

6.  The polypeptide $G_{505}$.

7.  The polypeptide $G_{427}$.

8.  The polypeptide of any of the claims 1-7 which is produced by a recombinant host cell.

9.  A DNA sequence which encodes the polypeptide of any of the claims 1-8.

10. A replicable expression vector capable, in a transformant recombinant host cell, of expressing the DNA sequence of claim 9.

0258L

11. The vector of claim 10 which is a plasmid.

12. A vector of claim 11 wherein the plasmid is selected from pRabGtrp¹, pRabdex2, pRabdex3l, pRabGT1, and pRabdex1.

13. A recombinant host cell transformed with the vector of any of claims 10-12.

14. A cell culture comprising the recombinant host cells of Claim 13.

15. A process for producing the expresion vector of claims 10 to 12 which comprises constructing a first DNA sequence encoding the polypeptide and operably linking said first DNA sequence to a second DNA sequence capable of effecting expression of said first DNA sequence.

16. A process for producing a polypeptide which is immunogenic for antibodies against rabies infection in mammals which process comprises:

    a) constructing a replicable expression vector comprising a first DNA sequence encoding said polypeptide operably linked to a second DNA sequence capable of effecting the expression of the first DNA sequence;

    b) transforming host cells with the vector of a);

    c) growing said host cells under conditions favorable to the expression of the first DNA sequence;

    d) recovering the polypeptide.

17. The polypeptide produced by the method of claim 16.

18. A rabies vaccine comprising the polypeptide of any one of claims 1 to 8.

```
         S1                              S10                             S19
         met val pro gln val leu leu phe val leu leu leu gly phe ser leu cys phe gly

1                              10                             20
lys phe pro ile tyr thr ile pro asp lys leu gly pro trp ser pro ile asp ile his his leu arg cys pro

              30                             40                             50
asn asn leu val val glu asp glu gly cys thr [asn leu ser] gly phe ser tyr met glu leu lys val gly tyr

                   60                             70
ile ser ala ile lys val asn gly phe thr cys thr gly val val thr glu ala glu thr tyr thr asn phe val

              80                             90                            100
gly tyr val thr thr thr phe lys arg lys his phe arg pro thr pro asp ala cys arg ala ala tyr asn trp

                        110                            120
lys met ala gly asp pro arg tyr glu glu ser leu gln asn pro tyr pro asp tyr his trp val arg thr val

              130                            140                            150
arg thr thr lys glu ser leu ile ile ile ser pro ser val thr asp leu asp pro tyr asp lys ser leu his

                        160                            170
ser arg val phe pro ser gly lys cys ser gly ile thr val ser ser thr tyr cys ser thr asn his asp tyr

              180                            190                            200
thr ile trp met pro glu asn pro arg pro gly thr pro cys asp ile phe thr asn ser arg gly lys arg ala

                        210                            220
ser asn gly [asn lys thr] cys gly phe val asp glu arg gly leu tyr lys ser leu lys gly ala cys arg leu

              230                            240                            250
lys leu cys gly val leu gly leu arg leu met asp gly thr trp val ala met gln thr ser asp glu thr lys

                        260                            270
trp cys ser pro asp gln leu val asn leu his asp phe arg ser asp glu ile glu his leu val val glu glu

              280                            290                            300
leu val lys lys arg glu glu cys leu asp thr leu glu ser ile met thr thr lys ser val ser phe arg arg

                        310                            320
leu ser his leu arg lys leu val pro gly phe gly lys ala tyr thr ile phe [asn lys thr] leu met glu ala

              330                            340                            350
asp ala his tyr lys ser val arg thr trp asn glu ile ile pro ser lys gly cys leu lys val gly gly arg

                        360                            370
cys his pro his val asn gly val phe phe asn gly ile ile leu gly pro asp asp arg val leu ile pro glu

              380                            390                            400
met gln ser ser leu leu arg gln his met glu leu leu glu ser ser val ile pro leu met his pro leu ala

                        410                            420
asp pro ser thr val phe lys glu gly asp glu ala glu asp phe val glu val his leu pro asp val tyr lys

              430                            440                            450
gln ile ser gly val asp leu gly leu pro asn trp gly lys ▒tyr val leu met thr ala gly ala met ile gly▒
  ↑Bgl II                                                                 └Pst I
                                     460                            470
▒leu val leu ile phe ser leu met thr trp cys▒ arg arg ala asn arg pro glu ser lys gln arg ser phe gly
                                                         ‾‾‾     ‾‾‾         ‾‾‾     ‾‾‾

              480                            490                            500
gly thr gly gly asn val ser val thr ser gln.ser gly lys val ile pro ser trp glu ser tyr lys ser gly
                     ‾‾‾                   ‾‾‾     ‾‾‾

              505
gly glu ile arg leu end
                  ‾‾‾
```

## Fig.1.

Fig.2.

*Fig. 3.*

# 0117657

European Patent Office

**EUROPEAN SEARCH REPORT**

Application number

| DOCUMENTS CONSIDERED TO BE RELEVANT | | | EP 84300627.1 |
|---|---|---|---|
| Category | Citation of document with indication, where appropriate, of relevant passages | Relevant to claim | CLASSIFICATION OF THE APPLICATION (Int. Cl. 3) |
| X,D | NATURE, vol. 294, no. 5838, 19.-25. November 1981, (New York, USA) A. ANILIONIS et al.: "Structure of the glycoprotein gene in rabies virus" pages 275-278 * Page 275; fig. 4 * | 1-9 | C 07 C 103/52 C 12 N 15/00 C 12 P 21/00 C 12 N 7/00 A 61 K 39/205 |
| A,D | DEVELOPMENTS IN BIOLOGICAL STANDARDIZATION, vol. 40, 1978, (Basel CH) B. DIETZSCHOLD et al.: "Structure and Function of Rabies Virus. Glycoprotein" pages 45-55 * Abstract * | 1-7 | |
| | | | **TECHNICAL FIELDS SEARCHED (Int. Cl. 3)** C 07 C 103/00 C 12 N C 12 P A 61 K |

The present search report has been drawn up for all claims

| Place of search | Date of completion of the search | Examiner |
|---|---|---|
| VIENNA | 30-04-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document